# EUROPÄISCHE PATENTSCHRIFT

(11) **EP 0 407 909 B1**
(45) Veröffentlichungstag und Bekanntmachung des Hinweises auf die Patenterteilung: **02.11.1994**
(21) Anmeldenummer: 90112919.7
(22) Anmeldetag: 06.07.1990
(51) Int. Cl.: C12P 41/00, C12P 7/40

(54) **Verfahren zur Herstellung von R-(+)-3-Oxocycloalkancarbonsäureniederalkylestern**
Process for the preparation of R-(+)-3-oxo cycloalkane carboxylic lower alkyl esters
Procédé de préparation d'esters alcoyliques inférieurs de l'acide R-(+)-3-oxocycloalkane carboxylique

(30) Priorität: 11.07.1989 DE 3922752
(43) Veröffentlichungstag der Anmeldung: 16.01.1991
(73) Patentinhaber: BOEHRINGER INGELHEIM KG, 55216 Ingelheim (DE); BOEHRINGER INGELHEIM INTERNATIONAL GmbH, 55218 Ingelheim am Rhein (DE)
(72) Erfinder: Schwall, Horst, Dipl.Chem. Dr., D-6535 Gau-Algesheim (DE); Sobotta, Rainer, Dipl.Chem. Dr., D-6507 Ingelheim am Rhein (DE)

(56) Entgegenhaltungen:
- TETRAHEDRON LETTERS, Nr. 34, 1973, Pergamon Press, GB; J.B. JONES et al., Seiten 3165-3168#
- TETRAHEDRON LETTERS, Band 29, Nr. 51, 1988, Pergamon Press, GB; T.H.D. BUGG et al., Seiten 6779-6782#
- TETRAHEDRON LETTERS, Band 29, Nr. 47, 1988, Pergamon Press, GB; F. TRIGALO et al., Seiten 6109-6112#

## Beschreibung

Die Erfindung betrifft ein Verfahren zur Herstellung von R-(+)-3-Oxocycloalkancarbonsäureniederalkylestern (wobei "Cycloalkan" für Cycloheptan und Cyclohexan und "Niederalkyl" für C₁-C₄-Alkyl steht) durch enzymatische Spaltung der entsprechenden Racemate.

Die erfindungsgemäß herstellbaren Enantiomeren sind wichtige Ausgangsprodukte für die Herstellung bestimmter bekannter, als Arzneistoffe verwendbarer Hetrazepine. Für die Synthese enantiomerenreiner Verbindungen aus dieser Substanzklasse ist die Verwendung von 3-Oxocycloalkancarbonsäureestern vorteilhaft, welche die gleiche Konfiguration am optisch aktiven Zentrum aufweisen wie der Wirkstoff selbst.

Es ist bekannt, 3-Oxocyclopentancarbonsäure mit Hilfe der optisch aktiven Base Brucin zu trennen (Bull. Chem. Soc. Jap. 31, 333-335 (1958)). Zur Herstellung des für die Hetrazepin-Synthese benötigten Esters kann die freigesetzte R-(+)-3-Oxocyclopentancarbonsäure dann nach üblichen Methoden in den Ester übergeführt werden.

Dieses Verfahren ist jedoch für die Übertragung in den technischen Maßstab wenig geeignet, weil Brucin sehr toxisch und schlecht verfügbar ist.

Aus TETRAHEDRON LETTERS, Nr. 34 (1993), S. 3165-3168, ist auch bekannt, daß R-(+)-3-oxocyclohexancarbonsäuremethylester durch Hydrolyse des Racemats mittels einer Hydrolase (α-Chymotrypsin) erhalten werden kann. Das genannte Enzym muß jedoch in relativ großer Menge angewendet werden, erfordert eine sehr lange Reaktionszeit und liefert ein Produkt, bei dem die Enantiomeren nur sehr unvollständig getrennt sind.

Gemäß der Erfindung können nun die R-(+)-3-Oxocycloalkancarbonsäureester mit guter Ausbeute in geeigneter Reinheit auch in technischem Maßstab erhalten werden, indem man racemische 3-Oxocycloalkancarbonsäureester mit Schweineleberesterase (PLE) behandelt, welche den nicht benötigten Ester spaltet, so daß die entstehende Säure leicht vom gewünschten Ester abgetrennt und dieser dann isoliert werden kann.

Das neue Verfahren wird zweckmäßig in einer Mischung aus Wasser und wassermischbaren Cosolventien wie Methanol, Dimethylformamid, Dimethylsulfoxid bei Temperaturen - zwischen etwa 0 und 40 vorzugsweise zwischen 5 und 30°C ausgeführt, wobei der pH Wert mittels eines üblichen Phosphatpuffers und Alkalizugabe zwischen etwa 7 und 8 gehalten wird.

Die als Hydrolase verwendete Schweineleberesterase (PLE) kann auch in an sich bekannter Weise immobilisiert angewendet werden, z. B. gebunden an das Handelsprodukt Eupergit C (Fa. Röhm Pharma, Darmstadt).

Die benötigte Menge Enzym beträgt etwa 20-200 u/g Substrat. Als Substrat können vor allem in Betracht die Niederalkylester, insbesondere der Methyl- oder Ethylester der 3-Oxocylcopentacarbonsäure und der 3-Oxocyclohexancarbonsäure.

Ein Beispiel für die Ausführung des Verfahrens, das gewünschtenfalls modifiziert werden kann, ist nachstehend angegeben.

### Herstellung des R-(+)-3-Oxocyclopentancarbonsäuremethylesters

Zu einer gerührten Suspension von 142,2 g (1 mol) racemischen 3-Oxocyclopentancarbonsäuremethylester in 1500 ml 9,1 molarem Phosphatpuffer, pH 7,5, (Kaliumdihydrogenphosphat, Dinatriumhydrogenphosphat) wird bei 20°C Schweineleberesterase (20.000 Einheiten) zugegeben. Durch Zudosieren von 1N-Natronlauge mittels Autotitrator wird die Protonenkonzentration (pH 7,5) konstant gehalten. Nach einem Verbrauch von 700 ml der 1N-Natronlauge wird die Hydrolyse durch Extraktion* abgebrochen. Der nicht umgesetzte Ester wird durch dreimaliges Ausrühren mit je 500 ml Methylenchlorid extrahiert. Die Methylenchloridphase wird über Natriumsulfat getrocknet, filtriert und das Lösungsmittel im Vakuum abdestilliert. Der Rückstand wird ohne Destillation weiterverarbeitet.
Ausbeute: 40 g (56,3 % d. Th.), [α]²⁰_{D} = + 27,44,
c= 1,618 (Methanol).
*Bei Verwendung immobilisierten Enzyms kann das Verfahren durch Abfiltrieren beendet werden.

Das Produkt ist für die Weiterverarbeitung ausreichend isomerenrein.

## Patentansprüche

1. Verfahren zur Herstellung von R-(+)-3-Oxocyclopentan- bzw. -hexancarbonsäure-C₁-C₄-alkylestern aus racemischen 3-Oxocyclopentan- bzw. hexancarbonsäure-C₁-C₄-alkylestern, dadurch gekennzeichnet, daß man das Racemat in einem wäßrigen Medium ggf. in Mischung mit Cosolventien wie Methanol, Dimethylformamid, Dimethylsulfoxid, bei Temperaturen zwischen etwa 0 und 40°C und bei einem pH-Wert zwischen etwa 7 und 8 mit Schweineleberesterase (PLE) behandelt, welche die Hydrolyse des unerwünschten Enantiomeren bewirkt, anschließend die Säure bzw. ihr Salz abtrennt und den R-(+)-3-Oxocycloalkancarbonsäureester isoliert.

2. Verfahren nach Anspruch 1, dadurch gekennzeichnet, daß als Ausgangsstoff racemischer 3-Oxocyclopentan- oder 3-Oxocyclohexancarbonsäuremethylester verwendet wird.

## Claims

1. A process of preparing R-(+)-3-oxocyclopentane- or -hexane carboxylic acid-C₁₋₄-alkylesters from racemic 3-oxocyclopentane- or hexane-carboxylic acid-C₁₋₄-alkylesters, characterized in that the racemate is treated in an aqueous medium, optionally mixed with cosolvents such as methanol, dimethylformamide, dimethylsulphoxide, at temperatures of between about 0 and 40°C and at a pH value of between about 7 and 8, using pigs liver esterase (PLE) which effects the hydrolysis of the undesired enantiomer, followed by the separation of the acid or the salt thereof and the isolation of the R-(+)-3-oxocycloalkanecarboxylate.

2. A process according to claim 1, characterised in that racemic 3-oxocyclopentane- or 3-oxocyclohexane carboxylic acid methyl ester is used as starting material.

## Revendications

1. Procédé de préparation d'esters d'alkyle en C₁-C₄ d'acide R-(+)-3-oxocyclopentanecarboxylique ou d'acide R-(+)-3-oxocyclohexanecarboxylique à partir d'esters d'alkyle en C₁-C₄ d'acide 3-oxocyclopentanecarboxylique ou d'acide 3-oxocyclohexanecarboxylique racémiques, caractérisé en ce que l'on traite le racémate dans un milieu aqueux, éventuellement en mélange avec des cosolvants comme le méthanol, le diméthylformamide, le diméthylsulfoxyde, à des températures comprises entre environ 0 et 40°C et à un pH compris entre environ 7 et 8 avec l'estérase de foie de porc (PLE) qui provoque l'hydrolyse de l'énantiomère indésirable, puis l'on sépare l'acide ou son sel et on isole l'ester d'acide R-(+)-3-oxocycloalcanecarboxylique.

2. Procédé selon la revendication 1, caractérisé en ce que l'on utilise comme produit de départ le 3-oxocyclopentanecarboxylate de méthyle racémique ou le 3-oxocyclohexanecarboxylate de méthyle racémique.
